# FASCICULE DE BREVET EUROPEEN

(11) **EP 0 419 541 B1**
(45) Date de publication et mention de la délivrance du brevet: **09.02.1994**
(21) Numéro de dépôt: 89907175.7
(22) Date de dépôt: 02.06.1989
(51) Int. Cl.: A61H 39/00, A61N 5/06

(54) **DISPOSITIF PODOLOGIQUE POUR LA CORRECTION DES TROUBLES ET DES AFFECTIONS DU TONUS NEUROMUSCULAIRE DE POSTURE**
PODOLOGIE-VORRICHTUNG ZUR KORREKTUR VON STÖRUNGEN UND SCHÄDIGUNGEN AM NEUROMUSKULÄREN TONUS DER HALTUNG
PODOLOGICAL DEVICE FOR CORRECTING DISORDERS AND AFFECTIONS OF THE POSTURAL NEUROMUSCULAR TONUS

(30) Priorité: 17.06.1988 FR 8808347
(43) Date de publication de la demande: 03.04.1991
(73) Titulaire: SOUVESTRE, Philippe, F-13010 Marseille (FR)
(72) Inventeur: SOUVESTRE, Philippe, F-13010 Marseille (FR)
(74) Mandataire: Moretti, René
(86) Numéro de dépôt international: FR8900275
(87) Numéro de publication internationale: WO8912435

(56) Documents cités:
- NL-A- 8 602 221

## Description

La présente invention a pour objet des dispositifs podologiques pour la correction des troubles et des affections du tonus neuromusculaire de posture.

Le secteur technique de l'invention est la fabrication de supports plantaires de type semelle et/ou matériel orthopédique.

Une des applications de l'invention est la réalisation d'un dispositif comprenant une semelle glissée dans chacune des chaussures ou l'une seulement, d'un patient pour l'amélioration de son équilibre postural et la restauration de sa capacité de correction spontanée.

On connaît en effet de nombreux dispositifs permettant le traitement de certains troubles pathologiques des organismes vivants par action externe sans pénétration matérielle d'instruments à l'intérieur de cesdits organismes, ni injection de substances chimiques

Des brevets ont été déposés depuis moins de 10 ans sur des dispositifs ou des procédés utilisant différentes sources d'actions externes telles que le rayonnement magnétique, électrique ou lumineux (laser, ultraviolets, infrarouge, lumière visible etc...).

On peut citer par exemple, par ordre chronologique, ceux qui sont les plus représentatifs de l'état de la technique en ALLEMAGNE et en FRANCE essentiellement,qui sont les pays les plus en pointe dans ce domaine, à savoir :
1 - Le brevet allemand du 29 Octobre 1975, étendu à la FRANCE le 25 Octobre 1976 sous le No. FR-A-2 371 935 par la Société MESSERSCHMITT-BOLKOW-BLOHM GESELLSCHAFT concernant un "appareil faisant appel à l'action de la lumière pour des traitements thérapeutiques semblables à l'acupuncture". Il s'agit en fait d'une projection de rayonnement laser de faible puissance interrompu de façon cyclique par un obturateur suivant une fréquence de 2 à 20 hz, stimulant des points précis (point lumineux de l'ordre de 1 mm de diamètre), déterminés en acupuncture et donnant les mêmes effets qu'avec des aiguilles.
2 - Le brevet français déposé le 24 Novembre 1976 sous le No. FR-A-2 329 258 par Monsieur P. NOGIER concernant un "procédé et appareil pour stimulation locale par rayonnement électromagnétique". Ce rayonnement est en fait produit par un faisceau au moins semi-cohérent, de rayons infrarouges,dont la fréquence de base est composée d'un premier terme à 73 hz, puis de tous les multiples en série géométrique de raison 2, et éclairant des points choisis du corps non précisés et sans donner de précision sur les effets obtenus.
3- Le brevet français déposé le 15 Février 1979 sous le NO. FR-A-2 453 654 par Monsieur P. NOGIER décrivant un "procédé et appareil pour le traitement magnétique d'organismes vivants". Il s'agit d'un dispositif propre à polariser la lumière, de type verre polaroïde, interposé entre une source de flux magnétique de type aimant et l'organisme à traiter; le flux polarisé pénètre alors profondément dans l'organisme sans perdre sa polarisation contrairement à la lumière polarisée. Les appareils utilisant ce procédé sont commercialisés sous le nom de "POLARTRON" et agissent en fait sur le système nerveux pour calmer les douleurs (oreilles, cicatrices etc...).
4 - Le brevet français déposé le 28 Octobre 1980 sous le NO. FR-A-2 492 666 par Monsieur Christian C. MARET concernant un "appareil à émission lumineuse ayant un effet physiologique par voie cutanée". Cet appareil est assez complexe, car il comprend un émetteur de lumière clignotante suivant une période de 2 à 6 secondes, traversant successivement et dans cet ordre un ensemble de filtres colorés, puis de couches de tissu imprégné de substances actives diluées. Ce dispositif est plutôt fixé au poignet ou à la cheville de l'utilisateur et permet de combiner plusieurs actions thérapeutiques par un choix approprié de ou des filtres associés aux substances actives diluées et dynamisées selon les méthodes d'HAHNEMANN.
5 - Le brevet français déposé le 13 Mai 1981 sous le NO. FR-A-2 505 660 et étendu à l'EUROPE sous le NO. EP-A-0 102 434 par Monsieur Bernard BRICOT, sur le quel se fonde le preambule des revendications, décrivant un "dispositif podal pour le traitement des zones réflexes des pieds et, notamment des troubles et affections du 7S au déséquilibre rachidien". Il s'agit ici de stimuler des zones réflexes des pieds, assez générales du reste puisque le dispositif revendiqué peut couvrir toute la surface plantaire,par un polariseur constitué par au moins deux plaques ou feuilles d'une matière ayant une orientation moléculaire ou cristalline déterminée, ces plaques étant superposées de telle façon que leur axe de polarisation soit croisé. Ce dispositif utilise en fait le même principe thérapeutique que le POLARTRON décrit dans le brevet de Monsieur NOGIER, mais sans source active magnétique qui est toxique si l'intensité est trop forte, surtout en utilisation podale. Les résultats thérapeutiques sont obtenus au bout de quelques mois par un rééquilibre du rachis et des améliorations de quelques perturbations fonctionnelles en rapport avec la statique.
6 - Le brevet allemand déposé le 22 Juillet 1983 sous le NO. P 33 26 513.5 et étendu à l'EUROPE dont la FRANCE, par Monsieur TETZNER Volkmar, concernant un "appareil d'irradiation pour des traitements photobiologiques et photochimiques". Cet appareil comprend une source d'éclairage ultraviolet éclairant la peau pour des traitements en particulier dermatologiques, à travers un jeu de filtres placés dans un boîtier, deux à deux, pouvant être interchangés facilement suivant le traitement.

La plupart de ces dispositifs ou procédés utilisent une source active externe, ce qui en complique l'utilisation (encombrement, poids) et en interdit le port permanent, car il faut une source d'énergie. De plus, leur action peut être toxique car l'intensité du rayonnement peut avoir un effet négatif sur l'organisme. Aucun de ces équipements n'a du reste des résultats statistiquement probants et réguliers; l'interposition de filtre (polarisant ou coloré) permet certes d'optimiser les effets positifs de ladite source, dont certains sont du reste utilisés directement sur l'organisme (laser, lampe ultraviolet, champ magnétique etc....).

Ces filtres sont rajoutés pour limiter en fait le rayonnement de cette source en ne laissant passer que la partie active du flux de celui-ci, qui est alors renforcé.

Par ailleurs, l'application de ces appareils se fait en des points qui ne sont jamais bien précisés par les inventeurs, qui ont en général, constaté des effets bénéfiques sur les organismes, mais sans résultats ni tests prouvés et décrits dans leur invention, car effectivement, ceux-ci peuvent être très différents d'une personne à l'autre et c'est l'expérience de l'utilisateur qui montre à ceux-ci les zones du corps les plus réceptives et les traitements résultants correspondants qu'il peut éventuellement en attendre.

Seule l'invention citée de Monsieur BRICOT précise son unique zone d'application sous la plante du pied, mais pouvant être quant même prise dans son ensemble, n'utilise pas de source active externe et donne des exemples d'améliorations thérapeutiques, mais sans les quantifier.

Celles-ci ont été constatées cependant depuis, et on a noté qu'il faut attendre jusqu'à six à dix mois pour obtenir un résultat probant et durable, avec un début de réaction au bout de quinze jours à un mois, et ceci seulement pour certaines affections de déséquilibres très particulières.

De plus, il faut que les plaques polarisantes soient pratiquement en contact direct avec le pied, et certains tissus, tel le nylon, arrêtent complètement leur effet; par ailleurs, la transpiration et le frottement dégradent ces plaques rapidement; or, il faut un port permanent pendant donc une année environ.

En effet, l'homme est un animal debout et le point d'appui de son corps est bien la plante du pied; des expérimentations ont démontré ainsi que les systèmes musculaires dits de posture se structurent en un certain nombre d'ensemble, regroupés en cinq chaînes : les deux médianes sont statiques (antéromédianes et postéromédianes), les deux chaînes latérales sont dynamiques et la dernière (antéro-postérieure) est régulatrice des quatre autres. Toutes ces chaînes musculaires ont un point de départ podal. La résultante de leur activité constitue, chez un sujet debout, immobile et au repos, regardant droit devant lui un point à l'infini, un état statique qui peut s'analyser dans les trois directions de l'espace et les trois plans géométriques qui les caractérisent : plan frontal, plan horizontal, plan sagittal médian.

Si l'on étudie globalement une population occidentale, on retrouve plus de 90% de troubles de la statique vertébrale, or il y a des relations étroites entre ceux-ci, les douleurs vertébrales, celles d'origine vertébrale, ou à composante statique, et les déformations de la colonne vertébrale.

En effet, la phylogénèse des espèce nous montre que l'acquisition de la station verticale chez l'homme est une véritable prouesse d'équilibre, difficilement maîtrisée encore et pour laquelle une année d'apprentissage après la naissance est nécessaire, ceci grâce à un certain nombre d'éléments coordonnés et asservis : le cerveau, les chaînes musculaires, les capteurs périphériques que sont notamment le pied (répartition géométrique des pressions sous la voûte plantaire) et l'oeil, et les voies proprioceptives entre autres qui relient ces différents éléments dans les deux sens.

On comprend ainsi qu'une action sur toute zone sensible du corps peut avoir des répercussions sur certains organes, grâce à ces voies proprioceptives et qu'une action plus particulière sous la voûte plantaire peut avoir un effet très important sur toute la statique corporelle et sur d'autres troubles de l'organisme concerné.

Ainsi, de nombreuses recherches ont été menées et se développent toujours en matière de traitement podologique et d'appareil de correction, en particulier sur les semelles proprioceptives telles que celles à champ polarisant, et on pourra se reporter à ce sujet à la conférence du Docteur MARIGNAN aux Journées Nationale de Podo-Orthèse des 1er et 2 Mai 1987 à MARSEILLE.

Il existe également des semelles dites classiques, qui comportent de grosses cales, pour chercher à corriger la déformation d'un pied, tel un pied Valgus. Dans ce cas, non seulement on ne corrige pas toujours le trouble statique correspondant, mais on risque de l'aggraver, car la réaction propagée le long des voies proprioceptives agit en rétro-action et lutte contre l'obstacle supplémentaire qui lui est imposé.

Enfin le Docteur BOURDIOL, a pour sa part, mis au moins un système de semelles proprioceptives où, par des mini reliefs, il induit des réflexes correcteurs. Ces reliefs n'ont plus pour but de basculer les pièces osseuses, mais d'enclencher des réflexes proprioceptifs de correction, par action au niveau cutané et musculaire.

Cependant pour être efficace, il faut que le patient soit toujours en appui sur ses deux semelles, ce qui est rarement le cas, et en cas d'appui unipodal, elles peuvent être dangereuses.

Le problème posé est de pouvoir corriger d'une façon permanente et assez rapidement par action externe tous les troubles et les affections du tonus neuromusculaire de posture sans limitation des origines et des causes, sans immobiliser le patient, ni gêner ses mouvements et déplacements, tout en soulageant son état général.

Une solution au problème posé est un dispositif podologique pour la correction des troubles et des affections du tonus neuromusculaire de posture, comportant au moins un film filtrant souple d'ondes électromagnétiques ayant une courbe de transmission spectrale déterminée : le dispositif suivant l'invention comprend soit deux films filtrants superposés tel que caractérisé dans la revendication 1, soit trois films filtrants superposés également et tel que caractérisé suivant la revendication 2, et en outre, une structure d'appui du pied placé toujours dans la même position relative par rapport à cette dite structure, et dans laquelle sont incorporés lesdits films filtrants à un emplacement situé en regard d'une zone-réflexe déterminée du pied.

Dans un mode de réalisation préférentiel, l'emplacement desdits films filtrants est en regard d'une zone antérieure réflexe de la partie plantaire du pied au niveau de l'un quelconque des espaces intermétatarsiens.

De même et de préférence, ladite structure d'appui comprend une semelle composée de divers éléments connus, par exemple assemblés en couches superposées, laquelle semelle incorpore dans l'un de ses éléments, lesdits films filtrants placés au plus près de la surface d'appui supérieure.

Le résultat est un nouveau dispositif podologique pour la correction des troubles et des affections du tonus neuromusculaires de posture. Les avantages d'un tel dispositif sont multiples, car il permet beaucoup de souplesse dans son utilisation et apporte une correction rapide dans certains troubles pathologiques, sans apport de produit ou d'énergie externe, donc sans risque de perturbation grave par ailleurs.

En effet, l'ensemble composé de filtres et de la structure d'appui, peut être une semelle souple, que l'utilisateur peut glisser dans ses chaussures normales. Les semelles pouvant être réalisées avec un revêtement de protection recouvrant le filtre, l'utilisateur peut commencer son traitement immédiatement lors de leur prescription et se déplacer avec, sans risque d'abîmer les filtres, qui conservent ainsi leur qualité. De plus, le patient peut garder n'importe quelle chaussette sans gêner l'action du dispositif.

Comme décrit ci-après, les effets du dispositif sont très rapidement perceptibles et soulagent les troubles à partir d'un délai de deux à dix jours environ, selon la gravité de la pathologie, au delà duquel on constate un changement de l'équilibre général.

La description suivante de ce dispositif se réfère aux dessins annexés qui représentent, sans aucun caractère limitatif, des exemples de réalisation du dispositif selon l'invention, qui sont donnés à titre d'illustration, mais d'autres réalisations utilisant en particulier d'autres filtres peuvent être envisagées.

Les figures 1 et 1a représentent un exemple de fabrication d'une semelle suivant l'invention en vue de dessus et en coupe.

La figure 2 montre une vue de dessous en transparence d'un dispositif en position relative par rapport à un pied s'appuyant dessus.

Les figures 3 et 4 représentent deux courbes de transmission spectrale de deux filtres différents.

La figure 5 représente deux filtres superposés avant assemblage dans le dispositif.

Les figures 6, 7 et 8 représentent trois courbes de transmission spectrale de trois filtres différents.

La figure 9 représente trois filtres superposés avant assemblage dans le dispositif.

La figure 10 représente un exemple de trouble statique dans le plan frontal.

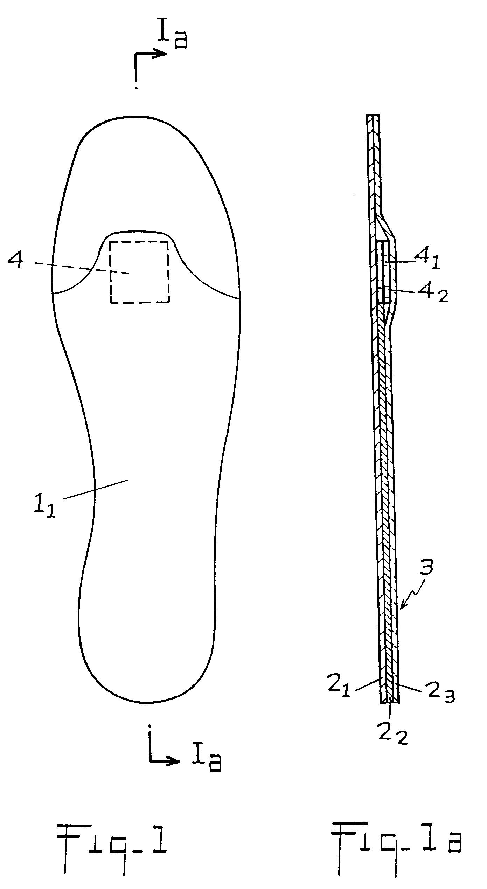

Les figures 1 et la représentent un exemple de réalisation d'un dispositif suivant l'invention. Il s'agit d'une structure d'appui faisant semelle 1₁, composée de différentes matières et éléments connus, figurés ici en trois couches superposées 2₁, 2₂ et 2₃, dont la dernière 2₃ est une couche de recouvrement dont la surface extérieure 3 est en contact avec la plante du pied.

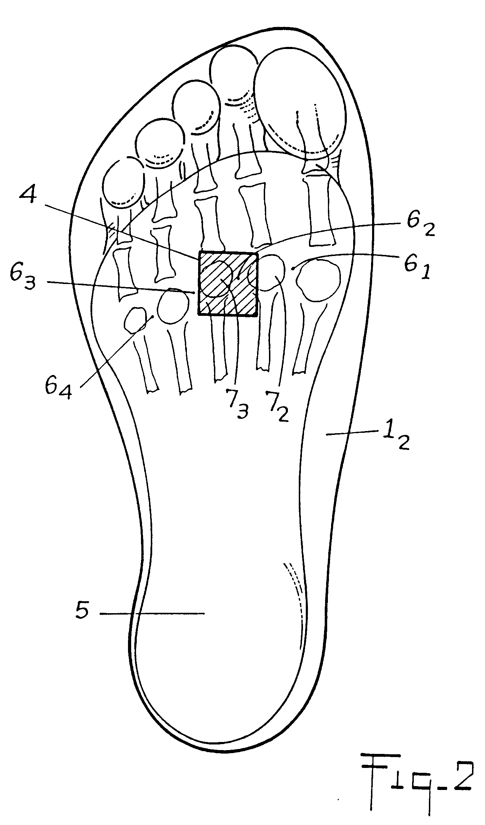

Sous cette dite couche de recouvrement 2₃, au moins un film filtrant 4 souple est incorporé dans ou sur la couche sous-jacente 2₃, en un emplacement disposé en regard d'une zone réflexe de la partie plantaire du pied tel que représenté dans la figure 2, et au plus près de la surface 3.

Ce film filtrant est représenté (figure 1a) en coupe de deux épaisseurs superposées 4₁ et 4₂, chacune correspondant par exemple à un filtre coloré mince, composé en particulier de pellicules de gélatine et de type de ceux utilisés pour les tirages photos et dont les courbes de transmission spectrale sont celles de la figure 3.

La surface de ce film filtrant 4 doit être supérieure à celle correspondant à la zone réflexe du pied en regard de laquelle ledit film est positionné et il est suffisant qu'elle soit légèrement supérieure, par exemple de l'ordre de 3 cm²; sa forme peut être quelconque, circulaire, rectangulaire ou carrée comme sur la figure 1.

Dans un mode d'utilisation préférentiel, la semelle 1₁ est glissée à l'intérieur d'une chaussure de marche traditionnelle, l'ensemble faisant alors une structure d'appui confortable pour l'usager et restant en permanence active au contact de la plante du pied, lequel pied pouvant être recouvert d'un tissu telle qu'une chaussette, sans gêner l'effet du dispositif.

La figure 2 est une vue de dessus en transparence d'un dispositif 1₂, suivant l'invention, par exemple de type semelle 1₁, telle que définie dans la figure 1, sur laquelle s'appuie un pied 5, toujours dans une même position relative nécessaire à l'efficacité du dispositif. Le film filtrant 4 doit être en effet en regard d'une zone réflexe déterminée du pied, notamment au niveau de l'un quelconque des espaces intermétatarsiens 6. Les quatre zones 6₁, 6₂, 6₃, 6₄ comprennent les muscles lombricaux et inter-osseux ainsi que les ramifications du fléchisseur commun des orteils et des troncs artériels.

En particulier, ledit film filtrant 4 est figuré en regard de la zone réflexe antérieure du pied, correspondant à l'espace intermétatartasien 6₂ situé entre la deuxième et la troisième têtes des métatarsiens 7₂ et 7₃. C'est sur cette zone que des dispositifs suivant l'invention,et incluant des films filtrants réalisés sur les figures 3 à 9,ont été testés et ont permis d'obtenir les résultats décrits dans la figure 10.

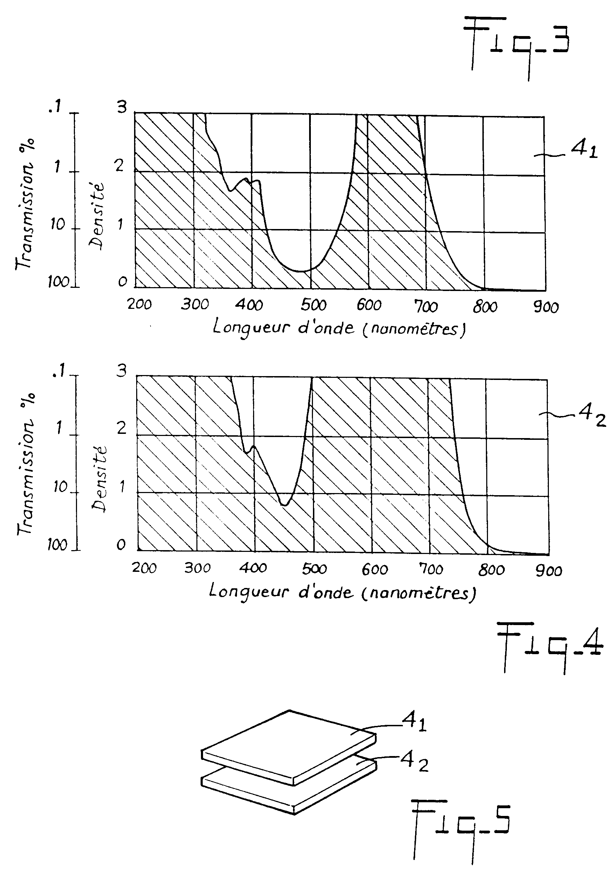

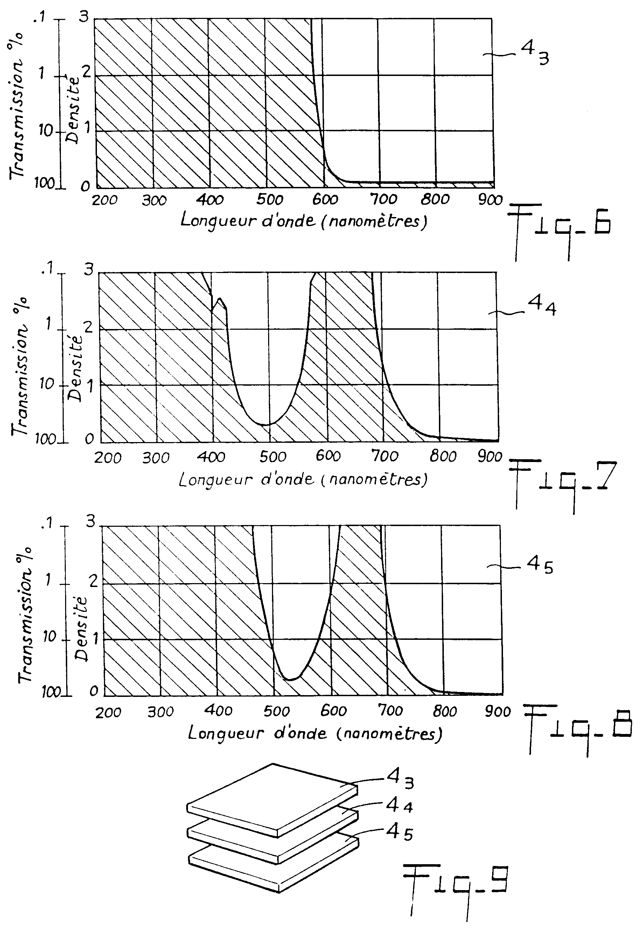

Les figures 3 et 4 représentent chacune la courbe de transmission spectrale de la lumière visible de deux films filtrants 4₁ et 4₂ connus et incorporés dans le dispositif suivant l'invention, tel que représenté dans la figure 1. Ces courbes donnent le pourcentage de transmission de la lumière de 1 à 100% en échelle logarithmique, en fonction de la longueur d'onde exprimée en nanomètres de 200 à 900 nanomètres.

La courbe de la figure 3 est celle d'un filtre de bandes passantes dans la lumière visible,d'une part comprise entre 320 et 580 nanomètres, avec un maximum de 53,3 % environ de transmission, entre 470 et 490 nanomètres et un minimum de 1,5% environ de transmission autour de 400 nanomètres, et d'autre part, au delà de 680 nanomètres.

La courbe de la figure 4 est celle d'un filtre de bandes passantes de la lumière visible, d'une part, comprise entre 360 et 490 nanomètres, avec un maximum de 18,2% de transmission environ autour de 450 nanomètres, et un minimum de 1,5 % de transmission environ autour de 400 nanomètres et, d'autre part, au delà de 730 nanomètres.

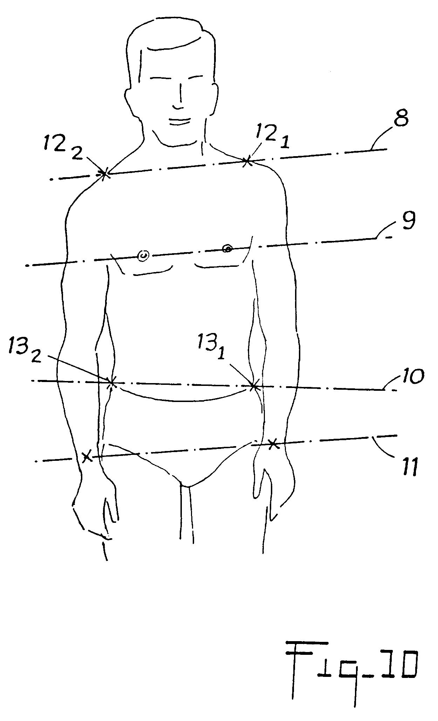

La figure 5 représente les deux films filtrants 4₁ et 4₂ tels que définis ci-dessus dans les figures 3 et 4, juste avant d'être superposés l'un contre l'autre, pour être incorporés par exemple dans une semelle tel que décrit dans la figure 1. Ces deux films peuvent être disposés indifféremment avec le film 4₁ ou 4₂ au-dessus.

Les figures 6, 7 et 8 représentent chacune la courbe de transmission spectrale de la lumière visible de trois films filtrants 4₃, 4₄ et 4₅ connus et incorporés dans le dispositif suivant l'invention, tel que représenté dans la figure 1. Les courbes donnent le pourcentage de transmission de la lumière de 1 à 100 % en échelle logarithmique, en fonction de la longueur d'onde exprimée en nanomètres de 200 à 900 nanomètres.

La courbe de la figure 6 est celle d'un filtre passe-haut de la lumière visible au delà de 690 nanomètres correspondant à une transmission minimum de 12,6 % environ.

La courbe de la figure 7 est celle d'un filtre de bandes passantes dans la lumière visible, d'une part, comprise entre 390 et 570 nanomètres, avec un maximum de 56,7% de transmission environ autour de 490 nanomètres, et un miminim de 0,27% environ de transmission autour de 410 nanomètres, et d'autre part, au delà de 690 nanomètres.

La courbe de la figure 8 est celle d'un filtre de bandes pasantes dans la lumière visible, d'une part comprise entre 470 et 610 nanomètres, avec un maximum de 53,6% de transmission environ autour de 530 nanomètres et, d'autre part au delà de 700 nanomètres.

La figure 9 représente les trois films filtrants 4₃, 4₄ et 4₅ tels que définis ci-dessus, juste avant d'étre superposés les uns contre les autres pour être incorporés par exemple dans une semelle tel que décrit dans la figure 1. Ces trois films doivent toujours être disposés dans l'ordre 4₃, 4₄ et 4₅, mais avec indifféremment 1e film 4₃ ou 4₅ au-dessus.

La figure 10 représente un exemple de trouble statique d'une personne dans le plan frontal, lesquels troubles sont corrigés par le port d'un dispositif suivant l'invention sous chaque pied.

Cela a été en particulier vérifié et prouvé en utilisant les combinaisons de filtres décrits dans les figures 3 à 9, disposés à l'emplacement de la zone réflexe 6₂ décrite dans la figure 2, lesquels filtres sont incorporés dans deux semelles 1₁, telles que décrites dans la figure 1 et sur lesquelles les patients prennent appui, en particulier après introduction dans leurs chaussures.

La personne représentée dans la figure 10 a toutes les lignes de ses ceintures basculées, ce qui est significatif de troubles statiques (ligne 8 des épaules, 9 des mamelons, 10 du bassin, 11 des poignets). Or on a constaté, après un port du dispositif suivant l'invention de quelques minutes et au maximum après 48 heures, une diminution significative de leur inclinaison, en particulier la ligne des épaules 8 acromio-claviculaire si elle est basculée jusqu'à 5 à 6 cm d'écart vertical entre les deux points 12₁ et 12₂, tend vers l'horizontale très rapidement et l'écart ci-dessus se réduit de 50% après 48 heures maximum, jusqu'à pratiquement s'annuler après plusieurs jours. Il en est de même pour la ligne 10 unissant les deux épines iliaques antéro-supérieures 13₁ et 13₂ si elle est basculée jusqu'à 2,5 cm d'écart vertical.

Ces résultats étaient totalement inattendus et sont le fruit de recherche et d'étude systématique, comparative et technique, auxquelles rien ne laisse prévoir à ce jour la raison ni l'explication, et aucune technique ni procédé antérieur ne pouvait orienter la recherche sur l'utilisation et l'action de ces filtres seuls et en indiquer une telle efficacité; celle-ci est donc tout à fait étonnante et de nombreux autres tests et constatations en apportent la confirmation dans le domaine de la statique de posture, mais également dans d'autres applications.

Sur des personnes ayant ainsi des troubles statiques dans le plan sagittal, se traduisant par une gêne dans l'amplitude rotatoire cervico céphalique, il a été constaté en particulier, un gain jusqu'à 10° dans la capacité de rotation de la tête après quelques minutes et au maximum 48 heures de port d'un dispositif suivant l'invention, et dans un des modes de réalisation cités dans le descriptif de la figure 10.

De même, avec les mêmes modes de réalisation, des personnes à jeun ont subi des tests de Fukuda et de Romberg qui sont respectivement :
- test de piétinement (une trentaine sur place), les yeux fermés, bras tendus en avant : la personne a tendance alors sans dispositif suivant l'invention à tourner autour de l'axe longitudinal de son corps vers la droite ou la gauche en rotation frontale. Avec le dispositif suivant l'invention, on constate une diminution de l'amplitude de rotation dans les quelsques minutes suivantes.
- test d'équilibre, les yeux fermés, les pieds joints parallèles, côte à côte ou l'un derrière l'autre, les bras le long du corps, la personne a tendance sans dispositif suivant l'invention à osciller et même dans certains cas à tomber. Avec le dispositif la personne reste debout et stable.

On note également des améliorations très surprenantes, qui laissent prévoir de nombreuses autres applications thérapeutiques du dispositif suivant l'invention avec les filtres et les emplacements définis ci-dessus 1 à 9, mais aussi avec d'autres choix de filtres, ayant d'autres courbures spectrales de transmission, et/ou placés en regard d'autres zones réflexes du pied, lesquelles améliorations portent pas exemple dans d'autres domaines médicaux tels que :
- en Ophtamologie, où l'on note une amélioration des troubles de vergence, c'est-à-dire par exemple des capacités de convergence et de divergence.
- en Stomatologie, où l'on remarque une amélioration des syndromes algodisfonctionnels de l'appareil manducateur (ou SADAM), c'est-à-dire des douleurs liées au trouble de fonctionnement de l'articulation temporo-mandibulaire.
- l'amélioration de l'état général de fatigue subjectif, du tonus émotionnel et des fonctions instrumentales telles que l'élocution, la mémoire, ou l'intelligibilité de situation.

L'utilisation du dispositif peut être faite également pour effectuer des diagnostics, car suivant les individus, la réaction à un dispositif comportant un choix de filtres déterminé ne sera pas le même et il faut changer suivant le cas ces filtres pour obtenir un meilleur résultat dans le domaine souhaité pour une personne donnée.

Il est important de noter que si les figures 3 et 4 et 6 à 8 représentent les courbes de transmission spectrale de la lumière visible des films filtrants cités à titre d'exemple, et ayant permis d'effectuer les tests décrits dans la figure 10 avec succès, cesdites courbes ne sont pas représentatives de la zone spectrale active de ces filtres pour obtenir les résultats suivant l'invention.

Il est même certain et prouvé que les fréquences actives effectives sont en dehors de la zone visible, mais nous avons représenté cette seule zone car elle est la plus couramment utilisée pour qualifier un filtre de par l'usage fait habituellement de ceux-ci dans le domaine photographique, et permet donc de définir ces exemples de filtres parmi ceux disponibles dans le commerce : il est cependant évident que d'autres filtres, ayant des courbes de transmission spectrale, dans le domaine du visible et même de l'invisible, différentes de ceux-ci, sont également efficaces et peuvent répondre au problème posé; leur utilisation dans le cadre de la présente invention est donc revendiquée.

Les tests ci-dessus sont cités en effet à titre d'exemple et de référence pour la quantification du dispositif suivant un mode de réalisation, mais d'autres modes de réalisation suivant l'invention sont possibles et la description des figures précédentes n'est pas limitative. L'intérêt du dispositif étant de pouvoir adapter à chaque personne et suivant ses troubles, un ensemble de filtres donnés superposés, dans un sens ou dans l'autre, et en un emplacement en regard d'une zone réflexe podale donnée, les domaines d'applications sont très ouverts et les choix de filtres non limitatifs, mais toujours à retenir suivant des critères bien précis.

Il ne faut pas confondre par ailleurs, ces divers filtres avec les films utilisés également en photographie, mais comme support de tirage des épreuves et qui sont donc sensibles à la lumière qui y provoque des réactions chimiques : ceux-ci sont par exemple utilisés et cités dans le brevet d'invention de Monsieur le Docteur T.S. TANG, déposé le 02 Septembre 1986 en HOLLANDE sous le NO. NL. A. 8.602.221 pour une action thérapeutique sur le visage, et sont donc de nature différente des films filtres dont il est question dans la présente invention.

## Revendications

1. Dispositif podologique pour la correction des troubles et des affections du tonus neuromusculaire de posture, comportant au moins un film filtrant souple d'ondes électromagnétiques, et une structure d'appui(1) du pied placé toujours dans la même position relative par rapport à cette dite structure, et dans laquelle sont incorporés les films filtrants en un emplacement situé en regard d'une zone déterminée du pied, caractérisé en ce que ledit dispositif comprend au moins deux films filtrants superposés de courbe de transmission spectrale dans des fréquences déterminées, l'un (4₁) ayant une courbe de transmission spectrale incluant des bandes passantes dans la lumière visible, d'une part comprise entre 320 et 580 nanomètres, avec un maximum de 53,3% environ de transmission entre 470 et 490 nanomètres et un minimum de 1,5 % environ de transmission autour de 400 nanomètres, et d'autre part, au delà de 680 nanomètres, l'autre (4₂) ayant une courbe de transmission spectrale incluant des bandes passantes de la lumière visible, d'une part comprise entre 360 et 490 nanomètres, avec un maximum de 18.2% de transmission environ autour de 450 nanomètres, et un minimum de 1,5% de transmission environ autour de 400 nanomètres et, d'autre part, au delà de 730 nanomètres, lesquels films (4₁ et 4₂) sont superposés indifféremment dans un ordre ou dans l'autre et placés en regard d'une zone-réflexe (6) déterminée du pied.

2. Dispositif podologique pour la correction des troubles et des affections du tonus neuromusculaire de posture, comportant au moins un film filtrant souple d'ondes électromagnétiques, et une structure d'appui(1) du pied placé toujours dans la même position relative par rapport à cette dite structure, et dans laquelle sont incorporés les films filtrants en un emplacement situé en regard d'une zone déterminée du pied, caractérisé en ce que ledit dispositif comprend au moins trois films filtrants superposés de courbe de transmission spectrale déterminée, l'un (4₃) ayant une courbe de transmission spectrale analogue à celle d'un filtre passe-haut de la lumière visible au delà de 690 nanomètres correspondant à une transmission minimum de 12,6% environ, un autre (4₄) ayant une courbe de transmission spectrale incluant des bandes passantes dans la lumière visible, d'une part, comprise entre 390 et 570 nanomètres, avec un maximum de 56,7% de transmission autour de 410 nanomètres, et d'autre part, au delà de 690 nanomètres et un autre (4₅) ayant une courbe de transmission spectrale incluant des bandes passantes dans la lumière visible, d'une part comprise entre 470 et 610 nanomètres, avec un maximum de 53,6% de transmission environ autour de 530 nanomètres et, d'autre part, au delà de 700 nanomètres, lesquels films filtrants (4₃, 4₄ et 4₅) sont superposés toujours dans ce même ordre, mais l'ensemble étant indifféremment tourné dans un sens ou dans l'autre et placé à un emplacement situé en regard d'une zone-réflexe (6) déterminée du pied.

3. Dispositif podologique suivant l'une des revendications 1 ou 2, caractérisé en ce que l'emplacement dudit film filtrant (4) est en regard d'une zone antérieure réflexe (6) de la partie plantaire du pied, au niveau de l'un quelconque des espaces intermétatarsiens (6₁, 6₂, 6₃, 6₄).

4. Dispositif podologique suivant la revendication 3, caractérisé en ce que ledit film filtrant (4) est en regard de la zone réflexe antérieure du pied, correspondant à l'espace intermétatarsien (6₂) situé entre la deuxième et la troisième tête de métatarsiens.

5. Dispositif podologique suivant l'une quelconque des revendications 1 à 4, caractérisé en ce que la structure d'appui comprend une semelle (1) composée de divers éléments connus par exemple assemblés en couches superposées (2), laquelle semelle incorpore dans l'un de ses éléments ledit film filtrant (4), placé au plus prés de la surface d'appui supérieure (3).

6. Dispositif podologique suivant la revendication 5, caractérisé en ce que la structure (1) est une chaussure de marche qui comprend ladite semelle (1₁).

7. Dispositif podologique suivant l'une quelconque des revendications 1 à 6, caractérisé en ce que ledit film filtrant (4) souple est un filtre coloré mince, composé de pellicules de gélatine.

8. Dispositif podologique suivant l'une quelconque des revendications 1 à 7, caractérisé en ce que le film filtrant (4) a une surface légèrement supérieure à celle correspondant à la zone réflexe du pied en regard de laquelle il est positionné.

## Claims

1. Podological device for correcting disorders and affections of the postural neuromuscular tonus, comprising at least one flexible filtering film for electromagnetic waves, and a support structure (1) for the foot which is always placed in the same relative position with respect to this said structure, and in which the filtering films are incorporated at a position situated opposite a defined zone of the foot, characterized in that the said device comprises at least two superposed filtering films having a spectral transmission curve in defined frequencies, one (4₁) having a spectral transmission curve including pass bands in visible light, on the one hand between 320 and 580 nanometres, with a maximum of approximately 53.3 % transmission between 470 and 490 nanometres and a minimum of approximately 1.5 % transmission in the region of 400 nanometres, and on the other hand beyond 680 nanometres, the other (4₂) having a spectral transmission curve including pass bands of visible light, on the one hand between 360 and 490 nanometres, with a maximum of approximately 18.2 % transmission in the region of 450 nanometres, and a minimum of approximately 1.5 % transmission in the region of 400 nanometres, and on the other hand beyond 730 nanometres, which films (4₁ and 4₂) are superposed equally well in one order or the other and are placed opposite a defined reflex zone (6) of the foot.

2. Podological device for correcting disorders and affections of the postural neuromuscular tonus, comprising at least one flexible filtering film for electromagnetic waves, and a support structure (1) for the foot which is always placed in the same relative position with respect to this said structure, and in which the filtering films are incorporated at a position situated opposite a defined zone of the foot, characterized in that the said device comprises at least three superposed filtering films having a defined spectral transmission curve, one (4₃) having a spectral transmission curve analogous to that of a high-pass filter for visible light beyond 690 nanometres corresponding to a minimum transmission of approximately 12.6 %, another (4₄) having a spectral transmission curve including pass bands in visible light, on the one hand between 390 and 570 nanometres, with a maximum of 56.7 % transmission in the region of 490 nanometres, and on the other hand beyond 690 nanometres, and another (4₅) having a spectral transmission curve including pass bands in visible light, on the one hand between 470 and 610 nanometres, with a maximum of approximately 53.6 % transmission in the region of 530 nanometres, and on the other hand beyond 700 nanometres, which filtering films (4₃, 4₄ and 4₅) are always superposed in this same order, but it being possible for the assembly to be turned equally well in one direction or the other and placed in a position situated opposite a defined reflex zone (6) of the foot.

3. Podological device according to either of Claims 1 and 2, characterized in that the position of the said filtering film (4) is opposite an anterior reflex zone (6) of the plantar part of the foot, at the level of any one of the intermetatarsal spaces (6₁, 6₂, 6₃, 6₄).

4. Podological device according to Claim 3, characterized in that the said filtering film (4) is opposite the anterior reflex zone of the foot, corresponding to the intermetatarsal space (6₂) situated between the second and third metatarsal heads.

5. Podological device according to any one of Claims 1 to 4, characterized in that the support structure comprises a sole (1) made up of various known elements, for example, assembled in superposed layers (2), which sole incorporates in one of its elements the said filtering film (4), placed nearest the upper support surface (3).

6. Podological device according to Claim 5, characterized in that the structure (1) is a walking shoe which comprises the said sole (1₁).

7. Podological device according to any one of Claims 1 to 6, characterized in that the said flexible filtering film (4) is a thin coloured filter made up of gelatin films.

8. Podological device according to any one of Claims 1 to 7, characterized in that the filtering film (4) has a surface slightly higher than that corresponding to the reflex zone of the foot opposite which it is positioned.

## Patentansprüche

1. Podologische Vorrichtung zur Korrektur von Störungen und Schädigungen am neuromuskulären Tonus der Haltung, mit wenigstens einem biegsamen, elektromagnetische Wellen filternden Film und einer Stützstruktur (1) für den Fuß, der immer in derselben Position relativ bezüglich dieser Struktur plaziert ist, und in der die filternden Filme an einer einer bestimmten Zone des Fußes gegenüberliegenden Stelle angebracht sind,
dadurch **gekennzeichnet,** daß
die Vorrichtung wenigstens zwei übereinandergelegte Filme mit spektraler Transmissionskurve bei gegebenen Frequenzen umfaßt, von denen einer (4₁) eine spektrale Transmissionskurve mit Durchgangsbändern im sichtbaren Bereich hat, einerseits zwischen 320 und 580 nm, mit einem Transmissionsmaximum von ca. 53,3 % zwischen 470 und 490 nm und einem Transmissionsminimum von ca. 1,5 % um 400 nm, und andererseits jenseits von 680 nm, und der andere (4₂) eine spektrale Transmissionskurve mit Durchgangsbändern im sichtbaren Bereich, einerseits zwischen 360 und 490 nm, mit einem Transmissionsmaximum von ca. 18,2 % um 450 nm und einem Transmissionsminimum von 1,5 % ungefähr um 400 nm und andererseits jenseits von 730 nm, welche Filme (4₁ und 4₂) in beliebiger Reihenfolge übereinander gegenüber einer bestimmten Reflexzone (6) des Fußes angeordnet sind.

2. Podologische Vorrichtung zur Korrektur von Störungen und Schädigungen am neuromuskulären Tonus der Haltung, mit wenigstens einem biegsamen, elektromagnetische Wellen filternden Film und einer Stützstruktur (1) für den Fuß, der immer in derselben Position bezüglich dieser Stützstruktur plaziert ist, und bei der die filternden Filme an einer einer bestimmten Zone des Fußes gegenüberliegenden Stelle angeordnet sind,
dadurch gekennzeichnet, daß
die Vorrichtung wenigstens drei übereinanderliegende Filme mit bestimmter spektraler Transmisslonskurve umfaßt, von denen einer (4₃) eine spektrale Transmissionskurve analog der eines Hochpaßfilters für sichtbares Licht jenseits von 690 nm mit einer minimalen Transmission von ca. 12,6 % hat, ein anderer (4₄) eine spektrale Transmissionskurve mit Durchgangsbändern im sichtbaren Bereich, einerseits zwischen 390 und 570 nm mit einem Transmissionsmaximum von 56,7 % um 490 nm und andererseits jenseits von 690 nm und ein anderer (4₅) eine spektrale Transmissionskurve mit Durchgangsbändern im sichtbaren Bereich, einerseits zwischen 470 und 610 nm mit einem Transmissionsmaximum von 53,6 % ungefähr um 530 nm und andererseits jenseits von 700 nm hat, welche filternden Filme (4₃, 4₄ und 4₅) immer in dieser Reihenfolge übereinander angeordnet sind, die Anordnung jedoch in eine oder die andere Richtung gedreht an einer Stelle gegenüber einer bestimmten Reflexzone (6) des Fußes plaziert ist.

3. Podologische Vorrichtung nach einem der Ansprüche 1 oder 2,
dadurch gekennzeichnet, daß
die Stelle des filternden Films (4) einer vorderen Reflexzone (6) des Sohlenbereichs des Fußes in Höhe eines beliebigen Mittelfußknochenzwischenraums (6₁, 6₂, 6₃ 6₄) liegt.

4. Podologische Vorrichtung nach Anspruch 3,
dadurch gekennzeichnet, daß
der filternde Film (4) der vorderen Reflexzone des Fußes, entsprechend dem zwischen der zweiten und der dritten Mittelfußknochenkuppe liegenden Mittelfußknochenzwischenraum, gegenüberliegt.

5. Podologische Vorrichtung nach einem der Ansprüche 1 bis 4,
dadurch gekennzeichnet, daß
die Stützstruktur eine Sohle (1) umfaßt, die aus unterschiedlichen bekannten Elementen besteht, die z.B. aus übereinander angeordneten Schichten (2) zusammengesetzt ist, wobei die Sohle in einem ihrer Elemente den filternden Film (4) so nahe wie möglich an der oberen Stützfläche (3) enthält.

6. Podologische Vorrichtung nach Anspruch 5,
dadurch gekennzeichnet, daß
die Struktur (1) ein Laufschuh ist, der die Sohle (1₁) umfaßt.

7. Podologische Vorrichtung nach einem der Ansprüche 1 bis 6,
dadurch gekennzeichnet, daß
der biegsame filternde Film (4) ein aus Gelatineplättchen zusammengesetzter dünner Farbfilter ist.

8. Podologische Vorrichtung nach einem der Ansprüche 1 bis 7,
dadurch gekennzeichnet, daß
der filternde Film (4) eine geringfügig höhere Oberfläche hat als die, die der Fußreflexzone, der gegenüber er positioniert ist, entspricht.
